(19) Europäisches Patentamt
European Patent Office
Office européen des brevets

(11) **EP 3 597 705 B1**

(12) **EUROPEAN PATENT SPECIFICATION**

(45) Date of publication and mention of the grant of the patent:
**03.08.2022 Bulletin 2022/31**

(21) Application number: **18767209.2**

(22) Date of filing: **13.03.2018**

(51) International Patent Classification (IPC):
***C08L 101/14*** (2006.01) ***A61F 13/15*** (2006.01)
***A61F 13/53*** (2006.01) ***A61L 15/20*** (2006.01)
***A61L 15/46*** (2006.01) ***A61L 15/50*** (2006.01)
***C08K 5/32*** (2006.01) ***C08J 3/12*** (2006.01)
***C08L 33/02*** (2006.01) ***C08L 33/12*** (2006.01)

(52) Cooperative Patent Classification (CPC):
(C-Sets available)
**C08J 3/128; A61F 13/15; A61F 13/53; A61L 15/20; A61L 15/46; A61L 15/50; C08K 5/32; C08L 101/14;**
C08J 2300/14 (Cont.)

(86) International application number:
**PCT/JP2018/009729**

(87) International publication number:
**WO 2018/168850 (20.09.2018 Gazette 2018/38)**

(54) **WATER ABSORBENT RESIN COMPOSITION, ABSORBENT, AND ABSORBENT ARTICLE**

WASSERABSORBIERENDE HARZZUSAMMENSETZUNG, SAUGFÄHIGES MATERIAL UND SAUGFÄHIGER ARTIKEL

COMPOSITION DE RÉSINE ABSORBANT L'EAU, ABSORBANT ET ARTICLE ABSORBANT

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **17.03.2017 JP 2017053599**

(43) Date of publication of application:
**22.01.2020 Bulletin 2020/04**

(73) Proprietor: **Sumitomo Seika Chemicals Co., Ltd.**
**Kako-gun Hyogo 675-0145 (JP)**

(72) Inventors:
- **MURAKAMI, Masahiro**
  **Himeji-shi**
  **Hyogo 672-8076 (JP)**
- **TANIMOTO, Daiki**
  **Himeji-shi**
  **Hyogo 672-8076 (JP)**

(74) Representative: **Mewburn Ellis LLP**
**Aurora Building**
**Counterslip**
**Bristol BS1 6BX (GB)**

(56) References cited:
**EP-A1- 1 616 581**
**WO-A2-98/26808**
**CN-B- 106 807 340**
**JP-A- S6 415 045**
**JP-A- 2001 258 934**
**JP-A- 2002 505 917**
**JP-A- 2012 153 823**
**JP-A- 2016 104 119**
**JP-A- 2017 177 065**
**US-A- 3 920 015**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 3 597 705 B1

(52) Cooperative Patent Classification (CPC): (Cont.)

C-Sets
**C08K 5/32, C08L 33/02;**
**C08L 101/14, C08K 5/32**

## Description

### TECHNICAL FIELD

The present invention relates to a water-absorbent resin composition, an absorbent material, and an absorbent article; more particularly, the present invention relates to a water-absorbent resin composition that constitutes an absorbent material suitably used for hygienic materials such as disposable diapers, sanitary napkins, and incontinence pads, and to an absorbent article using the absorbent material.

### BACKGROUND ART

In recent years, water-absorbent resins have been widely used in the field of hygienic materials such as disposable diapers, sanitary napkins, and incontinence pads.

As such water-absorbent resins, cross-linked products of partially neutralized acrylic acid polymers have been proposed as preferable water-absorbent resins. That is, the cross-linked products of partially neutralized acrylic acid polymers are resistant to decomposition or degradation; additionally, acrylic acid used as a raw material is readily industrially available, and thus, they can be produced at low cost with uniform quality.

An absorbent article such as a disposable diaper, a sanitary napkin, or an incontinence pad is composed of an absorbent material that absorbs and retains a body liquid such as urine or menses excreted from the body, the absorbent material being positioned mainly in a central portion, a liquid-permeable front sheet (top sheet) positioned on the side of the absorbent article that is brought into contact with the body, and a liquid-impermeable rear sheet (back sheet) positioned opposite to the side that is brought into contact with the body. The absorbent material is composed of hydrophilic fibers such as pulp and a water-absorbent resin.

When such an absorbent material is used for, for example, hygienic materials, unpleasant odors such as ammonia may be emitted from the absorbent material which has absorbed a body fluid, in particular, urine, blood, sweat and the like.

As a method of suppressing such an unpleasant odor, for example, there have been known a method of mixing an odor adsorbent such as an active carbon or zeolite with an adsorbent material (see, for example, Patent Documents 1 and 2), a method called masking in which an odor stronger than an unpleasant odorant is emitted from an absorbent material (see, for example, Patent Document 3), a method of chemically changing a component causing an unpleasant odor to a component which does not emit the unpleasant odor, and a method of mixing a heavy metal with an absorbent material to kill bacteria generating an unpleasant odor (see, for example, Patent Document 4).

On the other hand, when an absorbent material is manufactured using a water-absorbent resin, productivity may fall due to low flowability of the water-absorbent resin, and for example, there is a concern that the flowability of the water-absorbent resin may be further reduced depending on a component added when the method of suppressing an unpleasant odor is adopted.

JP 2001-258934 A concerns a water absorbent prepared by causing weakly acidic resin powder having a water absorbing property and a degree of neutralization of 40-65 mol% to contain or carry one, two, or more kinds of urease inhibiting agents and, as necessary, an organic acid (salt). EP 1616581 A1 concerns a water absorbent resin composition, comprising water absorbent resin particles having an internal cross-linked structure obtained by polymerizing a water-soluble unsaturated monomer, a nitrogenous ketone compound (containing no carboxyl group), and a bivalent and/or trivalent and/or tetravalent water-soluble metal salt, wherein a total amount of the nitrogenous ketone compound and the bivalent and/or trivalent and/or tetravalent water-soluble metal salt ranges from 0.01 to 100 parts by mass with respect to 100 parts by mass of the water absorbent resin particles. US 3920015 A concerns a diaper treated with a hydroxamic acid in an amount sufficient to inhibit the decomposition of urea to ammonia when wetted with urine. JP S64-15045 A concerns a urine absorbent wherein hydroxamic acid or a metal salt thereof is added in an amount of 0.001% (w/v) or more to perform polymerization. WO 98/26808 A2 concerns a combination of a material that inhibits the formation of odor that has at least one attribute selected from the group consisting of antimicrobial activity, urease inhibition activity, pH adjustment activity, and mixtures thereof, and an odor-absorbing material for objectionable odor molecules selected from the group consisting of cyclodextrin, zeolite, activated carbon, kieselguhr, acid salt forming materials and mixtures thereof.

PRIOR ART DOCUMENTS

PATENT DOCUMENTS

Patent Document 1: Japanese Patent Laid-open Publication No. 2001-37805
Patent Document 2: Japanese Patent Laid-open Publication No. 11-512946
Patent Document 3: Japanese Patent Laid-open Publication No. 6-51843
Patent Document 4: Japanese Patent Laid-open Publication No. 2001-505237

SUMMARY OF THE INVENTION

PROBLEMS TO BE SOLVED BY THE INVENTION

The present invention has a main object to provide a water-absorbent resin composition which is capable of suppressing unpleasant odors, while having excellent flowability.

MEANS FOR SOLVING THE PROBLEM

The inventors of the present invention conducted a diligent study to solve the aforementioned problem. As a result, it has been found that a water-absorbent resin composition containing a water-absorbent resin and a predetermined hydroxamic acid species or salt thereof can suppress unpleasant odors, and unexpectedly exhibits excellent flowability.
The present invention has been accomplished as a result of further study based on these findings.
In summary, the present invention provides aspects of the invention comprising the following features:

Item 1. A water-absorbent resin composition including

a water-absorbent resin in the form of particles and
a hydroxamic acid species represented by the following general formula (1) or an alkali metal salt thereof or an ammonium salt thereof:

[Chem. 1]

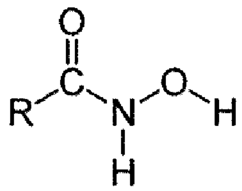

(1)

wherein a group R represents a phenyl group, a hydroxyphenyl group, a naphthyl group, a pyridyl group, an aromatic alkyl group having a butoxy group, alkyl group having a benzamide structure, or an alkyl group having a phenylcarbamoyl group, wherein the water-absorbent resin composition is a mixture of the hydroxamic acid species and the water-absorbent resin in the form of particles, and wherein the content of the hydroxamic acid species is in a range of 0.05 to 1.0 parts by mass per 100 parts by mass of the water-absorbent resin.

Item 2. The water-absorbent resin composition according to item 1, wherein the hydroxamic acid species represented by the general formula (1) is contained in the form of an alkali metal salt or an ammonium salt.
Item 3. The water-absorbent resin composition according to item 1 or 2, wherein the hydroxamic acid species or salt thereof is at least one selected from the group consisting of salicylhydroxamic acid, benzohydroxamic acid, 2-(4-butoxyphenyl) acetohydroxamic acid, 1-naphthohydroxamic acid, 3-pyridinecarbohydroxamic acid, suberoylanilide hydroxamic acid, 4-(dimethylamino)-N-[7-(hydroxyamino)-7-oxoheptyl]benzamide, and sodium benzohydroxamic acid.
Item 4. An absorbent material including the water-absorbent resin composition according to any one of items 1 to 3 and a hydrophilic fiber.
Item 5. An absorbent article including the absorbent material according to item 4 held between a liquid-permeable sheet and a liquid-impermeable sheet.

**ADVANTAGES OF THE INVENTION**

The present invention can provide a water-absorbent resin composition capable of suppressing unpleasant

odors and having excellent flowability. Furthermore, in accordance with the present invention, there is provided an absorbent article using the water-absorbent resin.

## EMBODIMENTS OF THE INVENTION

1. Water-Absorbent Resin Composition

The water-absorbent resin composition of the present invention is characterized by containing a water-absorbent resin and hydroxamic acid species represented by the following general formula (1) or salt thereof. Since the water-absorbent resin composition contains the hydroxamic acid species or salt thereof, even when the water-absorbent resin composition absorbs a component causing unpleasant odor, generation of the unpleasant odor (in particular, ammonia which is a typical component of unpleasant odor) can be effectively suppressed. Furthermore, since the water-absorbent resin composition contains the hydroxamic acid species or salt thereof, flowability of the water-absorbent resin composition is unexpectedly improved, and productivity of an absorbent material and the like using the water-absorbent resin composition can be improved. The water-absorbent resin composition of the present invention will be hereinafter described in detail.

(Hydroxamic Acid species)

The hydroxamic acid species contained in the water-absorbent resin composition of the present invention is represented by the following general formula (1) or an alkali metal salt thereof or an ammonium salt thereof:

[Chem. 2]

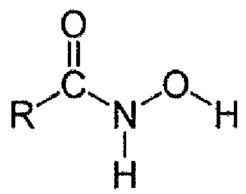

(1)

In the general formula (1), the group R represents a phenyl group, a hydroxyphenyl group, a naphthyl group, a pyridyl group, an aromatic alkyl group having a butoxy group, alkyl group having a benzamide structure, or an alkyl group having a phenylcarbamoyl group.

The hydroxamic acid species of the general formula (1) may be contained in the water-absorbent resin composition of the present invention in the form of a salt such as an alkali metal salt or an ammonium salt.

Specific preferred examples of hydroxamic acid species or salts thereof include salicylhydroxamic acid, benzohydroxamic acid, 2-(4-butoxyphenyl) acetohydroxamic acid, 1-naphthohydroxamic acid, 3-pyridinecarbohydroxamic acid, suberoylanilide hydroxamic acid, 4-(dimethylamino)-N-[7-(hydroxyamino)-7-oxoheptyl]benzamide, and sodium benzohydroxamic acid. In particular, from the viewpoint of easy availability, preferred are salicylhydroxamic acid. One or more types of the hydroxamic acid species and salts thereof may be contained in the water-absorbent resin composition of the present invention.

In the water-absorbent resin composition of the present invention, a blending ratio of hydroxamic acid species or salt thereof and the water-absorbent resin (total ratio in the case of containing plural types of hydroxamic acid species or salt thereof) is in a range of 0.05 to 1.0 parts by mass of the hydroxamic acid species per 100 parts by mass of the water-absorbent resin. From the viewpoint of further enhancing the effect of suppressing unpleasant odor of the water-absorbent resin composition and flowability of the water-absorbent resin composition while securing sufficient water-absorption capacity, a lower limit of the content of hydroxamic acid species or salt thereof is 0.05 parts by mass or more relative to 100 parts by mass of the water-absorbent resin. From the viewpoint of industrial ease of production and cost, an upper limit of the content of hydroxamic acid species or salt thereof is 1.0 part by mass or less relative to 100 parts by mass of the water-absorbent resin.

The water-absorbent resin composition of the present invention can be conveniently produced by mixing the hydroxamic acid species or salt thereof with the water-absorbent resin.

(Water-Absorbent Resin)

The water-absorbent resin contained in the water-absorbent resin composition of the present invention is formed from, for example, a polymer of a water-soluble ethylenically unsaturated monomer.

The water-absorbent resin is usually in the form of particles. The water-absorbent resin preferably has a median particle diameter of 200 to 600 μm, more preferably 250 to 500 μm, and still more preferably 300 to 450 μm.

The particles of the water-absorbent resin may be in the form in which each the particle is formed with a single particle or may be in the form (secondary particles) in which minute particles (primary particles) are agglomerated. Examples of the shape of the primary particle include a substantially spherical shape, a crushed indefinite shape, and a flat shape. When the particles of the water-absorbent resin are primary particles manufactured by reverse phase suspension polymerization, it is possible to produce a water-absorbent resin having a substantially spherical single-particle shape that has a smooth surface shape such as a spherical shape or an elliptical shape. Since the surface shape is smooth in the primary particles with such a shape to increase the flowability of the powder thereof, and the agglomerated particles are easily and densely packed, the secondary particles are unlikely to be broken by a shock. Therefore, the water-absorbent resin having a high particle strength is easily obtained.

The median particle diameter of the water-absorbent resin can be measured using JIS standard sieves. More specifically, the median particle diameter represents a value as measured using the method described in the Examples.

The water-absorbent resin may contain additives suitable for its purpose. Examples of such additives include inorganic powders, surfactants, oxidizing agents, reducing agents, metal chelating agents, radical chain inhibitors, antioxidants, anti-bacterial agents, and deodorizers. For example, when 0.05 to 5 parts by mass of amorphous silica as an inorganic powder is added to 100 parts by mass of the water-absorbent resin, the flowability of the water-absorbent resin can be further improved.

To polymerize the water-soluble ethylenically unsaturated monomer, a representative polymerization method such as aqueous solution polymerization, emulsion polymerization, or reversed phase suspension polymerization is used. In aqueous solution polymerization, polymerization is performed by heating, optionally with stirring, an aqueous solution of the water-soluble ethylenically unsaturated monomer. In reversed phase suspension polymerization, polymerization is performed by heating the water-soluble ethylenically unsaturated monomer with stirring in a hydrocarbon dispersion medium. In the present invention, reversed phase suspension polymerization is preferred from the viewpoint of allowing the polymerization reaction to be precisely controlled, and a wide range of particle diameters to be controlled.

One exemplary method for producing the water-absorbent resin will be hereinafter described.

Specific examples of methods for producing the water-absorbent resin include a method for producing the water-absorbent resin by performing reversed phase suspension polymerization of the water-soluble ethylenically unsaturated monomer in a hydrocarbon dispersion medium, the method including the steps of: performing the polymerization in the presence of a radical polymerization initiator; and post-crosslinking the hydrous gel obtained by the polymerization in the presence of a post-crosslinking agent. In the method for producing the water-absorbent resin of the present invention, an internal-crosslinking agent may be added, as required, to the water-soluble ethylenically unsaturated monomer to obtain a hydrous gel having an internal-crosslinked structure.

<Polymerization Step>

[Water-Soluble Ethylenically Unsaturated Monomer]

Examples of the water-soluble ethylenically unsaturated monomer include (meth)acrylic acid ("acryl" and "methacryl" are herein collectively referred to as "(meth)acryl"; the same applies below) and salts thereof; 2-(meth)acrylamido-2-methylpropanesulfonic acid and salts thereof; nonionic monomers such as (meth)acrylamide, N,N-dimethyl(meth)acrylamide, 2-hydroxyethyl(meth)acrylate, N-methylol(meth)acrylamide, and polyethylene glycol mono(meth)acrylate; and amino group-containing unsaturated monomers such as N,N-diethylaminoethyl(meth)acrylate, N,N-diethylaminopropyl(meth)acrylate, and diethylaminopropyl(meth)acrylamide, as well as quaternary compounds thereof. Preferred among these water-soluble ethylenically unsaturated monomers are (meth)acrylic acid and salts thereof, (meth)acrylamide, and N,N-dimethyl(meth)acrylamide, and more preferred are (meth)acrylic acid and salts thereof, from the viewpoint of being readily industrially available. These water-soluble ethylenically unsaturated monomers may be used alone or in combination of two or more.

Among these water-soluble ethylenically unsaturated monomers, acrylic acid and salts thereof are widely used as raw materials of water-absorbent resins. Copolymers of acrylic acid and/or salts thereof with other water-soluble ethylenically unsaturated monomers as mentioned above may also be used. In this case, an acrylic acid and/or a salt thereof as a main water-soluble ethylenically unsaturated monomer is preferably used in an amount of 70 to 100 mol% based on the total amount of water-soluble ethylenically unsaturated monomers.

The water-soluble ethylenically unsaturated monomer is preferably dispersed as an aqueous solution in a

hydrocarbon dispersion medium, and then subjected to reversed phase suspension polymerization. When the water-soluble ethylenically unsaturated monomer is in the form of an aqueous solution, the dispersion efficiency in the hydrocarbon dispersion medium can be increased. The concentration of the water-soluble ethylenically unsaturated monomer in the aqueous solution is preferably in the range of 20% by mass to not more than the saturation concentration. The concentration of the water-soluble ethylenically unsaturated monomer is more preferably 55% by mass or less, still more preferably 50% by mass or less, and even more preferably 45% by mass or less. On the other hand, the concentration of the water-soluble ethylenically unsaturated monomer is more preferably 25% by mass or more, still more preferably 28% by mass or more, and even more preferably 30% by mass or more.

When the water-soluble ethylenically unsaturated monomer has an acid group such as (meth)acrylic acid or 2-(meth)acrylamido-2-methylpropanesulfonic acid, the acid group may be neutralized with an alkaline neutralizing agent, as required, before use. Examples of such alkaline neutralizing agents include alkali metal salts such as sodium hydroxide, sodium carbonate, sodium hydrogen carbonate, potassium hydroxide, and potassium carbonate; and ammonia. These alkaline neutralizing agents may be used in the form of aqueous solutions to facilitate the neutralization operation. The above-mentioned alkaline neutralizing agents may be used alone or in combination of two or more.

The degree of neutralization of the water-soluble ethylenically unsaturated monomer with an alkaline neutralizing agent, calculated as the degree of neutralization of all acid groups in the water-soluble ethylenically unsaturated monomer, is preferably 10 to 100 mol%, more preferably 30 to 90 mol%, still more preferably 40 to 85 mol%, and even more preferably 50 to 80 mol%.

[Radical Polymerization Initiator]

Examples of the radical polymerization initiator to be added in the polymerization step include persulfates such as potassium persulfate, ammonium persulfate, and sodium persulfate; peroxides such as methyl ethyl ketone peroxide, methyl isobutyl ketone peroxide, di-t-butyl peroxide, t-butyl cumyl peroxide, t-butyl peroxyacetate, t-butyl peroxyisobutyrate, t-butyl peroxypivalate, and hydrogen peroxide; and azo compounds such as 2,2'-azobis(2-amidinopropane) dihydrochloride, 2,2'-azobis[2-(N-phenylamidino)propane] dihydrochloride, 2,2'-azobis[2-(N-allylamidino)propane] dihydrochloride, 2,2'-azobis{2-[1-(2-hydroxyethyl)-2-imidazolin-2-yl]propane} dihydrochloride, 2,2'-azobis{2-methyl-N-[1,1-bis(hydroxymethyl)-2-hydroxyethyl]propionamide}, 2,2'-azobis[2-methyl-N-(2-hydroxyethyl)-propionamide], and 4,4'-azobis(4-cyanovaleric acid). Preferred among these radical polymerization initiators are potassium persulfate, ammonium persulfate, sodium persulfate, and 2,2'-azobis(2-amidinopropane) dihydrochloride, from the viewpoint of being readily available and easy to handle. These radical polymerization initiators may be used alone or in combination of two or more. The above-mentioned radical polymerization initiators may also be used in combination with reducing agents such as sodium sulfite, sodium hydrogensulfite, ferrous sulfate, and L-ascorbic acid to be used as redox polymerization initiators.

The amount of the radical polymerization initiator to be used may be, for example, 0.00005 to 0.01 mol per mole of the water-soluble ethylenically unsaturated monomer, although not limited thereto. The use of the radical polymerization initiator in the above-defined range of amounts can avoid the occurrence of an abrupt polymerization reaction, and can complete the polymerization reaction in an appropriate time.

[Internal-Crosslinking Agent]

Examples of the internal-crosslinking agent include those that can crosslink the polymer of the water-soluble ethylenically unsaturated monomer to be used, for example: unsaturated polyesters obtained by reacting polyols such as diols and triols, e.g., (poly)ethylene glycol ["(poly)" means both cases with and without the prefix "poly"; the same applies below], (poly)propylene glycol, 1,4-butanediol, trimethylolpropane, and (poly)glycerin, with unsaturated acids such as (meth)acrylic acid, maleic acid, and fumaric acid; bisacrylamides such as N,N-methylenebisacrylamide; di or tri(meth)acrylic acid esters obtained by reacting polyepoxides with (meth)acrylic acid; carbamyl di(meth)acrylates obtained by reacting polyisocyanates such as tolylene diisocyanate and hexamethylene diisocyanate with hydroxyethyl (meth)acrylate; compounds having two or more polymerizable unsaturated groups such as allylated starch, allylated cellulose, diallyl phthalate, N,N',N"-triallylisocyanate, and divinylbenzene; polyglycidyl compounds such as diglycidyl compounds and triglycidyl compounds, e.g., (poly)ethylene glycol diglycidyl ether, (poly)propylene glycol diglycidyl ether, and (poly)glycerin diglycidyl ether; epihalohydrin compounds such as epichlorohydrin, epibromohydrin, and $\alpha$-methyl-epichlorohydrin; compounds having two or more reactive functional groups such as isocyanate compounds, e.g., 2,4-tolylene diisocyanate and hexamethylene diisocyanate; and oxetane compounds such as 3-methyl-3-oxetanemethanol, 3-ethyl-3-oxetanemethanol, 3-butyl-3-oxetanemethanol, 3-methyl-3-oxetaneethanol, 3-ethyl-3-oxetaneethanol, and 3-butyl-3-oxetaneethanol. Among these internal-crosslinking agents, polyglycidyl compounds are preferably used, diglycidyl ether compounds are more preferably used, and (poly)ethylene glycol diglycidyl ether, (poly)propylene glycol diglycidyl ether, and (poly)glycerin diglycidyl ether are still more preferably used. These internal-crosslinking agents may

be used alone or in combination of two or more.

The amount of the internal-crosslinking agent to be used is preferably 0.000001 to 0.02 mol, more preferably 0.00001 to 0.01 mol, still more preferably 0.00001 to 0.005 mol, and even more preferably 0.00005 to 0.002 mol, per mole of the water-soluble ethylenically unsaturated monomer.

[Hydrocarbon Dispersion Medium]

Examples of the hydrocarbon dispersion medium include $C_{6-8}$ aliphatic hydrocarbons such as n-hexane, n-heptane, 2-methylhexane, 3-methylhexane, 2,3-dimethylpentane, 3-ethylpentane, and n-octane; alicyclic hydrocarbons such as cyclohexane, methylcyclohexane, cyclopentane, methylcyclopentane, trans-1,2-dimethylcyclopentane, cis-1,3-dimethylcyclopentane, and trans-1,3-dimethylcyclopentane; and aromatic hydrocarbons such as benzene, toluene, and xylene. Among these hydrocarbon dispersion media, n-hexane, n-heptane, and cyclohexane, which are readily industrially available, stable in quality, and inexpensive, are particularly suitably used. These hydrocarbon dispersion media may be used alone or in combination of two or more. Examples of mixtures of hydrocarbon dispersion media include commercially available products such as Exxsol Heptane (from Exxon Mobil Corporation; containing 75 to 85% by mass of heptane and its isomeric hydrocarbons). The use of such a commercially available product also leads to favorable results.

The amount of the hydrocarbon dispersion medium to be used is preferably 100 to 1500 parts by mass, and more preferably 200 to 1400 parts by mass, per 100 parts by mass of the first-stage water-soluble ethylenically unsaturated monomer, from the viewpoint of homogeneously dispersing the water-soluble ethylenically unsaturated monomer, and facilitating control of the polymerization temperature. As described below, reversed phase suspension polymerization is performed in a single stage or two or more multiple stages. The first-stage polymerization as mentioned above refers to the first-stage polymerization reaction in single-stage polymerization or multi-stage polymerization (the same applies below).

[Dispersion Stabilizer]

(Surfactant)

In reversed phase suspension polymerization, a dispersion stabilizer may be used to improve the dispersion stability of the water-soluble ethylenically unsaturated monomer in the hydrocarbon dispersion medium. A surfactant may be used as such a dispersion stabilizer.

Examples of the surfactant include sucrose fatty acid esters, polyglycerin fatty acid esters, sorbitan fatty acid esters, polyoxyethylene sorbitan fatty acid esters, polyoxyethylene glycerin fatty acid esters, sorbitol fatty acid esters, polyoxyethylene sorbitol fatty acid esters, polyoxyethylene alkyl ethers, polyoxyethylene alkyl phenyl ethers, polyoxyethylene castor oil, polyoxyethylene hydrogenated castor oil, alkyl allyl formaldehyde condensate polyoxyethylene ethers, polyoxyethylene-polyoxypropylene block copolymers, polyoxyethylene polyoxypropyl alkyl ethers, polyethylene glycol fatty acid esters, alkyl glucosides, N-alkyl glyconamides, polyoxyethylene fatty acid amides, polyoxyethylene alkylamines, polyoxyethylene alkyl ether phosphates, and polyoxyethylene alkyl allyl ether phosphates. Among these surfactants, sorbitan fatty acid esters, polyglycerin fatty acid esters, and sucrose fatty acid esters, are particularly preferably used, from the viewpoint of dispersion stability of the monomer. These surfactants may be used alone or in combination of two or more.

The amount of the surfactant to be used is preferably 0.1 to 30 parts by mass, and more preferably 0.3 to 20 parts by mass, per 100 parts by mass of the first-stage water-soluble ethylenically unsaturated monomer.

(Polymeric dispersion agent)

A polymeric dispersion agent may be used in combination with the above-described surfactant, as a dispersion stabilizer to be used in reversed phase suspension polymerization.

Examples of the polymeric dispersion agent include maleic anhydride modified polyethylene, maleic anhydride modified polypropylene, maleic anhydride modified ethylene-propylene copolymers, maleic anhydride modified EPDM (ethylene-propylene-diene terpolymers), maleic anhydride modified polybutadiene, maleic anhydride-ethylene copolymers, maleic anhydride-propylene copolymers, maleic anhydride-ethylene-propylene copolymers, maleic anhydride-butadiene copolymers, polyethylene, polypropylene, ethylene-propylene copolymers, oxidized polyethylene, oxidized polypropylene, oxidized ethylene-propylene copolymers, ethylene-acrylic acid copolymers, ethyl cellulose, and ethyl hydroxyethyl cellulose. Among these polymeric dispersants, maleic anhydride modified polyethylene, maleic anhydride modified polypropylene, maleic anhydride modified ethylene-propylene copolymers, maleic anhydride-ethylene copolymers, maleic anhydride-propylene copolymers, maleic anhydride-ethylene-propylene copolymers, polyethylene, polypropylene, ethylene-propylene copolymers, oxidized polyethylene, oxidized polypropylene, and oxidized ethylene-propylene copolymers are particularly preferably used, from the viewpoint of dispersion stability of the monomer. These polymeric dispersion agent may be used alone or in combination of two or more.

The amount of the polymeric dispersion agent to be used is preferably 0.1 to 30 parts by mass, and more preferably 0.3 to 20 parts by mass, per 100 parts by mass of the first-stage water-soluble ethylenically unsaturated monomer.

[Other Components]

In the method for producing the water-absorbent resin, other components may be added, as desired, to the aqueous solution containing the water-soluble ethylenically unsaturated monomer to be subjected to reversed phase suspension polymerization. Various additives such as thickeners and chain transfer agents may be added as other components.

[Reversed Phase Suspension Polymerization]

To perform reversed phase suspension polymerization, for example, the aqueous monomer solution containing the water-soluble ethylenically unsaturated monomer is dispersed in a hydrocarbon dispersion medium, in the presence of a dispersion stabilizer. Here, so long as the dispersion stabilizer (a surfactant or a polymeric dispersion agent) is added before the beginning of the polymerization reaction, it may be added either before or after the aqueous monomer solution is added.

In particular, from the viewpoint of readily reducing the amount of remaining hydrocarbon dispersion medium in the resulting water-absorbent resin, it is preferred to disperse the aqueous monomer solution in the hydrocarbon dispersion medium in which a polymeric dispersion agent is dispersed, followed by dispersing a surfactant therein, and then perform polymerization.

Such reversed phase suspension polymerization can be performed in a single stage or two or more multiple stages. From the viewpoint of enhancing productivity, reversed phase suspension polymerization is preferably performed in two or three stages.

Reversed phase suspension polymerization with two or more multiple stages may be performed as follows: the first-stage reversed phase suspension polymerization is performed; subsequently, a water-soluble ethylenically unsaturated monomer is added to the reaction mixture obtained by the first-stage polymerization reaction and mixed, and reversed phase suspension polymerization in the second and subsequent stages is performed in the same manner as in the first stage. In reversed phase suspension polymerization in each of the second and subsequent stages, in addition to the water-soluble ethylenically unsaturated monomer, a radical polymerization initiator is preferably added within the above-described range of molar ratios of each of the components relative to the water-soluble ethylenically unsaturated monomer, based on the amount of the water-soluble ethylenically unsaturated monomer added during reversed phase suspension polymerization in each of the second and subsequent stages. In the second and subsequent stages of polymerization, an internal-crosslinking agent may also be added, as required, to the water-soluble ethylenically unsaturated monomer.

The reaction temperature during the polymerization reaction is preferably 20 to 110°C, and more preferably 40 to 90°C, from the viewpoint of allowing the polymerization to proceed quickly to reduce the polymerization time for improved economical efficiency, and readily removing the heat of polymerization to perform a smooth reaction.

<Post-Crosslinking Step>

A water-soluble ethylenically unsaturated monomer is polymerized through the above-mentioned <Polymerization Step>, and a hydrous gel having an internal-crosslinked structure is obtained. The hydrous gel may be subjected as it is to <Drying Step> described later, or may be post-crosslinked with a post-crosslinking agent (that is, may be subjected to the post-crosslinking reaction) as described in this section. The post-crosslinking reaction is preferably preformed in the presence of a post-crosslinking agent, after the polymerization of the water-soluble ethylenically unsaturated monomer. When the hydrous gel having an internal-crosslinked structure is thus subjected to the post-crosslinking reaction after the polymerization, a water-absorbent resin can be achieved in which the crosslinking density in the vicinity of the surface has been increased to improve various kinds of performance such as the water-absorption capacity under a load.

Examples of the post-crosslinking agent include compounds having two or more reactive functional groups. Examples of the post-crosslinking agent include polyols such as ethylene glycol, propylene glycol, 1,4-butanediol, trimethylolpropane, glycerin, polyoxyethylene glycol, polyoxypropylene glycol, and polyglycerin; polyglycidyl compounds such as (poly)ethylene glycol diglycidyl ether, (poly)glycerin diglycidyl ether, (poly)glycerin triglycidyl ether, trimethylolpropane triglycidyl ether, (poly)propylene glycol polyglycidyl ether, and (poly)glycerol polyglycidyl ether; haloepoxy compounds such as epichlorohydrin, epibromohydrin, and α-methylepichlorohydrin; isocyanate compounds such as 2,4-tolylene diisocyanate and hexamethylene diisocyanate; oxetane compounds such as 3-methyl-3-oxetanemethanol, 3-ethyl-3-oxetanemethanol, 3-butyl-3-oxetanemethanol, 3-methyl-3-oxetaneethanol, 3-ethyl-3-oxetaneethanol, and 3-butyl-3-oxetaneethanol; oxazoline compounds such as 1,2-ethylenebisoxazoline; carbonate compounds such as ethylene carbonate; and hydroxyalkylamide compounds such as bis[N,N-di(β-hydroxyethyl)]adipamide. Preferred among these post-crosslinking agents are polyglycidyl compounds such as (poly)ethylene glycol diglycidyl ether, (poly)glycerin diglycidyl ether, (poly)glycerin triglycidyl ether, trimethylolpropane triglycidyl ether, (poly)propylene glycol polyglycidyl ether, and (poly)glycerol polyglycidyl ether. These post-crosslinking agents may be used alone or in combination of two or more.

The amount of the post-crosslinking agent to be used is preferably 0.00001 to 0.01 mol, more preferably 0.00005 to 0.005 mol, and still more preferably 0.0001 to 0.002 mol, per mole of the water-soluble ethylenically unsaturated monomer subjected to polymerization. In the case of multistage reversed-phase suspension polymerization by two or more stages, the amount of the water-soluble ethylenically unsaturated monomer, which is the basis of the amount of the post-crosslinking agent to be used, is the total amount of the water-soluble ethylenically unsaturated monomer used in each stage.

The post-crosslinking agent may be added as is or as an aqueous solution. As required, a solution of the post-crosslinking agent in a hydrophilic organic solvent may be added. Examples of such hydrophilic organic solvents include lower alcohols such as methyl alcohol, ethyl alcohol, n-propyl alcohol, and isopropyl alcohol; ketones such as acetone and methyl ethyl ketone; ethers such as diethyl ether, dioxane, and tetrahydrofuran; amides such as N,N-dimethylformamide; and sulfoxides such as dimethylsulfoxide. These hydrophilic organic solvents may be used alone, in combination of two or more, or as a mixture with water.

The post-crosslinking agent may be added after the polymerization reaction of the water-soluble ethylenically unsaturated monomer is substantially completed. The post-crosslinking agent is preferably added in the presence of 1 to 400 parts by mass of water, more preferably 5 to 200 parts by mass of water, still more preferably 10 to 100 parts by mass of water, and even more preferably 20 to 60 parts by mass of water, per 100 parts by mass of the water-soluble ethylenically unsaturated monomer. The amount of water herein refers to the total amount of the water contained in the reaction system and the water that is used, as required, during the addition of the post-crosslinking agent.

The reaction temperature during the post-crosslinking reaction is preferably 50 to 250°C, more preferably 60 to 180°C, still more preferably 60 to 140°C, and even more preferably 70 to 120°C. The reaction time of the post-crosslinking reaction is preferably 1 to 300 minutes, and more preferably 5 to 200 minutes.

<Drying Step>

After reversed phase suspension polymerization described above, a drying step of adding external energy such as heat to remove water, hydrocarbon dispersion medium, and the like out of the system by distillation may be performed. To remove the water in the hydrous gel after reversed phase suspension polymerization, the system in which the hydrous gel is dispersed in the hydrocarbon dispersion medium is heated to distill the water and the hydrocarbon dispersion medium out of the system by azeotropic distillation. Here, if the distilled hydrocarbon dispersion medium only is returned into the system, continuous azeotropic distillation can be performed. In this case, the temperature within the system during drying is maintained at a temperature not higher than the azeotropic temperature with the hydrocarbon dispersion medium, which is preferable from the viewpoint of inhibiting deterioration of the resin. Subsequently, the water and the hydrocarbon dispersion medium are distilled off to obtain particles of the water-absorbent resin. By controlling the treatment conditions for the drying step after the polymerization to adjust the amount of water to be removed, various kinds of performance of the resulting water-absorbent resin can be controlled.

In the drying step, the drying treatment by distillation may be performed under atmospheric pressure or reduced pressure. The drying treatment may also be performed in a stream of nitrogen or the like, from the viewpoint of enhancing the drying efficiency. When the drying treatment is performed under atmospheric pressure, the drying temperature is preferably 70 to 250°C, more preferably 80 to 180°C, still more preferably 80 to 140°C, and even more preferably 90 to 130°C. When the drying treatment is performed under reduced pressure, the drying temperature is preferably 40 to 160°C, and more preferably 50 to 110°C.

When the post-crosslinking step with a post-crosslinking agent is performed after the polymerization of the monomer by reversed phase suspension polymerization, the drying step by distillation is preferably performed as described above, after the completion of the post-crosslinking step. Alternatively, the post-crosslinking step and the drying step may be performed simultaneously.

Furthermore, various additives such as chelating agents, reducing agents, oxidizing agents, anti-bacterial agents, and deodorizers may be added, as required, to the water-absorbent resin, after polymerization, during drying, or after drying.

2. Absorbent Material and Absorbent Article

The water-absorbent resin composition of the present invention constitutes an absorbent material to be used for hygienic materials such as sanitary items and disposable diapers, and is suitably used for an absorbent article including the absorbent material.
Here, the absorbent material including the water-absorbent resin composition of the present invention contains the water-absorbent resin composition of the present invention and hydrophilic fibers. Examples of structures of the absorbent material include a mixed dispersion obtained by mixing the water-absorbent resin composition and hydrophilic fibers to give a homogeneous composition; a sandwich structure in which the water-absorbent resin is sandwiched between layered hydrophilic fibers; and a structure in which the water-absorbent resin composition and hydrophilic fibers are wrapped in tissue paper. The absorbent material may also contain other components such as thermally fusible synthetic fibers for enhancing the shape retention properties of the absorbent material, a hot melt adhesive, and an adhesive binder such as an adhesive emulsion.
The content of the water-absorbent resin composition in the absorbent material is preferably 5 to 95% by mass, more preferably 20 to 90% by mass, and still more preferably 30 to 80% by mass.
Examples of hydrophilic fibers include cellulose fibers such as cotton-like pulp made from wood, mechanical pulp, chemical pulp, and semi-chemical pulp; artificial cellulose fibers such as rayon and acetate; and fibers made of synthetic resins such as hydrophilized polyamide, polyester, and polyolefin.
The absorbent material including the water-absorbent resin composition of the present invention can be held between a liquid-permeable sheet (top sheet) that allows a liquid to pass through and a liquid-impermeable sheet (back sheet) that does not allow a liquid to pass through, to obtain an absorbent article of the present invention. The liquid-permeable sheet is positioned on the side of the absorbent article that is brought into contact with the body, and the liquid-impermeable sheet is positioned opposite to the side that is brought into contact with the body.
Examples of the liquid-permeable sheet include air-through, spunbond, chemical bond, or needle punch non-woven fabrics made of fibers of polyethylene, polypropylene, polyester, or the like, and porous synthetic resin sheets. Examples of the liquid-impermeable sheet include synthetic resin films made of resins such as polyethylene, polypropylene, and polyvinyl chloride.

EXAMPLES

Hereinafter, the present invention will be described in detail with reference to examples and comparative examples. Examples 1 to 4, 9, 11 and 12 are not according to the invention.
Water-absorbent resins obtained in the following examples and comparative examples were evaluated using the tests described below. Each of the testing methods for evaluation will be hereinafter described.

<Median Particle Diameter>

JIS standard sieves having mesh sizes of 850 μm, 600 μm, 500 μm, 425 μm, 300 μm, 250 μm, and 150 μm, and a receptacle were combined in that order from the top.
50 g of the water-absorbent resin was placed on the top sieve of the combined sieves, and shaken for 20 minutes with a Ro-Tap shaker to conduct classification. After the classification, the particle size distribution was determined by calculating the mass of the water-absorbent resin remaining on each sieve as the mass percentage relative to the total mass. With regard to this particle size distribution, the mass percentage of the water-absorbent resin remaining on each sieve was integrated in descending order of particle diameter. Thereby, the relationship between the sieve mesh size and the integrated value of the mass percentage of the water-absorbent resin remaining on each sieve was plotted on logarithmic probability paper. The plots on the probability paper were connected with straight lines, and a particle diameter equivalent to 50% by mass of the integrated mass percentage was determined as the median particle diameter.

<Water Content>

About 2 g of the water-absorbent resin was precisely weighed out (mass Wa (g) of the water-absorbent resin before drying) in a previously weighed aluminum foil case (No. 8). The above sample was dried for 2 hours with a hot-air dryer (from ADVANTEC) set at an internal temperature of 105°C. Thereafter, the dried sample was allowed to be cooled in a desiccator, and the mass Wb (g) of the water-absorbent resin after drying was measured. The water content of the water-absorbent resin was calculated by the following equation:

$$\text{water content (\%)} = [[Wa - Wb]/Wa] \times 100$$

<Ammonia Generation Suppression Test>

Artificial urine was prepared by dissolving 25.0 g of urea, 9.0 g of sodium chloride, 0.6 g of magnesium sulfate (heptahydrate), 0.7 g of calcium lactate, 4.0 g of potassium sulfate, and 2.5 g of ammonium sulfate in 1.0 kg of distilled water. Also, 1.0 mL of urease (1000 U/ml of a 50% glycerin solution originating from Sword Bean (Canavalia gladiata) manufactured by MERCK) was diluted with distilled water 500 times to prepare a urease solution. 1.0 g of each sample (water-absorbent resin compositions of Examples 1 to 8 and a water-absorbent resin of Comparative Example 1) was placed in a sterile petri dish and added with a test liquid (prepared by mixing 30.0 g of the artificial urine with 1.0 mL of the above urease solution) to swell the sample. After the test liquid was added, the sample was sealed in a 2 L Tedlar bag, air in the bag was evacuated, and instead of the air, 900 mL of dry air was added into the bag. Subsequently, storage was performed at 30°C, and after 24 hours, the amount of generated ammonia was measured using a gas detecting tube (from Gastec Corporation, ammonia 3L, 3La, 3M). The measurement value (200 ppm) is taken as 100.0% (generation amount of Comparative Example 1), and the results are shown in Table 1.

<Flowability Test>

A spatula angle was measured with a powder tester PT-N (from Hosokawa Micron Corporation), and the flowability of the water-absorbent resin compositions of Examples 3 and 7 as representative examples and the water-absorbent resin of Comparative Example 1 was evaluated. The spatula angle is an inclination angle of a side of a resin powder deposited on a spatula, refers to an angle required to move the powder in a stationary state, and is one of indicators of flowability. It means that the smaller the value of the spatula angle, the better the flowability. The measurement procedure was performed according to an instruction of the powder tester. The measured values of the spatula angle are shown in Table 2.

<Production of Water-Absorbent Resin>

(Production Example 1)

A 2 L cylindrical round-bottomed separable flask equipped with a stirrer, two sets of paddle blades, a reflux condenser, a dropping funnel, and a nitrogen gas inlet tube was provided. This flask was charged with 340.0 g of n-heptane, and then 0.92 g of a sucrose stearate (Ryoto sugar ester S-370 from Mitsubishi-Kagaku Foods Corporation) and 0.92 g of a maleic anhydride-modified ethylene-propylene copolymer (Hi-wax 1105A from Mitsui Chemicals, Inc.) were added thereto. The mixture was heated with stirring to 80°C to dissolve the surfactant, and then cooled to 50°C.

Separately, 92.0 g (1.02 mol) of an 80% by mass aqueous solution of acrylic acid was placed in 500-mL Erlenmeyer flask, and 146.0 g of a 21% by mass aqueous solution of sodium hydroxide was added dropwise with external cooling to accomplish 75 mol% neutralization. Then, 0.11 g (0.41 mmol) of potassium persulfate as a radical polymerization initiator and 8.10 mg (0.046 mmol) of ethylene glycol diglycidyl ether as an internal-crosslinking agent were added and dissolved. As a result, a first-stage aqueous monomer solution was prepared.

The entire amount of the first-stage aqueous monomer solution was added to the separable flask, and the inside of the system was sufficiently replaced with nitrogen. Thereafter, the flask was immersed in a water bath at 70°C to raise the temperature, and the first-stage polymerization was carried out for 30 minutes, thereby obtaining a first-stage reaction mixture.

Separately, 128.8 g (1.43 mol) of an 80% by mass aqueous solution of acrylic acid was placed in another 500-mL Erlenmeyer flask, and 159.0 g of a 27% by mass aqueous solution of sodium hydroxide was added dropwise with external cooling to accomplish 75 mol% neutralization. Then, 0.16 g (0.59 mmol) of potassium persulfate as a radical polymerization initiator and 6.62 mg (0.038 mmol) of ethylene glycol diglycidyl ether as an internal-crosslinking agent were added and dissolved. As a result, a second-stage aqueous monomer solution was prepared.

The first-stage reaction mixture was cooled to 28°C, the second-stage aqueous monomer solution at the same temperature was added into the system, and the flask was kept at 25°C for 30 minutes while the internal of the system was replaced with nitrogen. Thereafter, the flask was again immersed in a water bath kept at 70°C to raise the temperature, and the second-stage polymerization was carried out for 30 minutes.

After the second-stage polymerization, the temperature of the reaction mixture was raised in an oil bath at 125°C to distill 248.0 g of water out of the system while refluxing n-heptane by azeotropic distillation of water and n-heptane. Then, 4.42 g (0.51 mmol) of a 2% by mass aqueous solution of ethylene glycol diglycidyl ether was added, and the post-crosslinking reaction was carried out at 80°C for 2 hours. Thereafter, the temperature of the reaction mixture

was raised in an oil bath at 125°C, and n-heptane was evaporated and dried to obtain 230.5 g of a water-absorbent resin. The median particle diameter of the water-absorbent resin was 400 μm, and the water content of the water-absorbent resin was 8%.

(Example 1)

Acetohydroxamic acid as a hydroxamic acid species was added to and mixed with the water-absorbent resin obtained in Production Example 1 in an amount of 0.05 parts by mass per 100 parts by mass of the water-absorbent resin to produce a water-absorbent resin composition.

(Example 2)

A water-absorbent resin composition was produced in the same manner as in Example 1 except that the addition amount of acetohydroxamic acid was 0.1 parts by mass per 100 parts by mass of the water-absorbent resin.

(Example 3)

A water-absorbent resin composition was produced in the same manner as in Example 1 except that the addition amount of acetohydroxamic acid was 0.5 parts by mass per 100 parts by mass of the water-absorbent resin.

(Example 4)

A water-absorbent resin composition was produced in the same manner as in Example 1 except that the addition amount of acetohydroxamic acid was 1.0 part by mass per 100 parts by mass of the water-absorbent resin.

(Example 5)

Salicylhydroxamic acid as a hydroxamic acid species was added to and mixed with the water-absorbent resin obtained in Production Example 1 in an amount of 0.05 parts by mass per 100 parts by mass of the water-absorbent resin to produce a water-absorbent resin composition.

(Example 6)

A water-absorbent resin composition was produced in the same manner as in Example 5 except that the addition amount of salicylhydroxamic acid was 0.1 parts by mass per 100 parts by mass of the water-absorbent resin.

(Example 7)

A water-absorbent resin composition was produced in the same manner as in Example 5 except that the addition amount of salicylhydroxamic acid was 0.5 parts by mass per 100 parts by mass of the water-absorbent resin.

(Example 8)

A water-absorbent resin composition was produced in the same manner as in Example 5 except that the addition amount of salicylhydroxamic acid was 1.0 part by mass per 100 parts by mass of the water-absorbent resin.

(Example 9)

Octanohydroxamic acid as a hydroxamic acid species was added to and mixed with the water-absorbent resin obtained in Production Example 1 in an amount of 0.5 parts by mass per 100 parts by mass of the water-absorbent resin to produce a water-absorbent resin composition.

(Example 10)

Benzohydroxamic acid as a hydroxamic acid species was added to and mixed with the water-absorbent resin obtained in Production Example 1 in an amount of 0.5 parts by mass per 100 parts by mass of the water-absorbent resin to produce a water-absorbent resin composition.

(Example 11)

Sodium acetohydroxamic acid as a hydroxamic acid species was added to and mixed with the water-absorbent resin obtained in Production Example 1 in an amount of 0.5 parts by mass per 100 parts by mass of the water-absorbent resin to produce a water-absorbent resin composition.

(Example 12)

Sodium octanohydroxamic acid as a hydroxamic acid species was added to and mixed with the water-absorbent resin obtained in Production Example 1 in an amount of 0.5 parts by mass per 100 parts by mass of the water-absorbent resin to produce a water-absorbent resin composition.

(Example 13)

Sodium benzohydroxamic acid as a hydroxamic acid species was added to and mixed with the water-absorbent resin obtained in Production Example 1 in an amount of 0.5 parts by mass per 100 parts by mass of the water-absorbent resin to produce a water-absorbent resin composition.

(Comparative Example 1)

The water-absorbent resin produced in Production Example 1 was used as it is as the water-absorbent resin of Comparative Example 1.

[Table 1]

| | Hydroxamic acid species | | Ammonia generation amount (%) after 24 hours (Ammonia generation amount of Comparative Example 1: 100%) |
|---|---|---|---|
| | Type | Addition amount (part(s) by mass) | |
| Comparative Example 1 | - | - | 100.0 |
| Example 1 | Acetohydroxamic acid | 0.05 | 15.0 |
| Example 2 | | 0.1 | 7.5 |
| Example 3 | | 0.5 | 1.0 |
| Example 4 | | 1.0 | 0.3 |
| Example 5 | Salicylhydroxamic acid | 0.05 | 40.0 |
| Example 6 | | 0.1 | 32.5 |
| Example 7 | | 0.5 | 1.8 |
| Example 8 | | 1.0 | 1.0 |
| Example 9 | Octanohydroxamic acid | 0.5 | 0.3 |
| Example 10 | Benzohydroxamic acid | 0.5 | 0.5 |
| Example 11 | Sodium acetohydroxamic acid | 0.5 | 2.5 |
| Example 12 | Sodium octanohydroxamic acid | 0.5 | 0.5 |
| Example 13 | Sodium benzohydroxamic acid | 0.5 | 0.5 |

[Table 2]

| | Flowability test (Spatula angle °) |
|---|---|
| Comparative Example 1 | 48.2 |
| Example 3 | 33.8 |
| Example 7 | 35.6 |

## Claims

1. A water-absorbent resin composition comprising:

   a water-absorbent resin in the form of particles; and
   a hydroxamic acid species represented by the following general formula (1) or an alkali metal salt thereof or an ammonium salt thereof:

   [Chem. 1]

   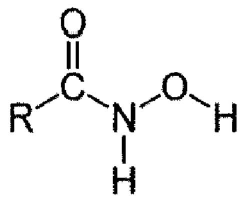

   (1)

   wherein a group R represents a phenyl group, a hydroxyphenyl group, a naphthyl group, a pyridyl group, an aromatic alkyl group having a butoxy group, alkyl group having a benzamide structure, or an alkyl group having a phenylcarbamoyl group, wherein the water-absorbent resin composition is a mixture of the hydroxamic acid species and the water-absorbent resin in the form of particles, and wherein the content of the hydroxamic acid species is in a range of 0.05 to 1.0 parts by mass per 100 parts by mass of the water-absorbent resin.

2. The water-absorbent resin composition according to claim 1, wherein the hydroxamic acid species represented by the general formula (1) is contained in the form of an alkali metal salt or an ammonium salt.

3. The water-absorbent resin composition according to claim 1 or 2, wherein the hydroxamic acid species or salt thereof is at least one selected from the group consisting of salicylhydroxamic acid, benzohydroxamic acid, 2-(4-butoxyphenyl) acetohydroxamic acid, 1-naphthohydroxamic acid, 3-pyridinecarbohydroxamic acid, suberoylanilide hydroxamic acid, 4-(dimethylamino)-N-[7-(hydroxyamino)-7-oxoheptyl]benzamide, and sodium benzohydroxamic acid.

4. An absorbent material comprising the water-absorbent resin composition according to any one of claims 1 to 3 and a hydrophilic fiber.

5. An absorbent article comprising the absorbent material according to claim 4 held between a liquid-permeable sheet and a liquid-impermeable sheet.

## Patentansprüche

1. Wasserabsorbierende Harzzusammensetzung, die Folgendes umfasst:

   ein wasserabsorbierendes Harz in Form von Teilchen; und
   eine Hydroxamsäure-Spezies, die durch die folgende allgemeine Formel (1) dargestellt ist, oder ein Alkalimetallsalz davon oder ein Ammoniumsalz davon:

[Chem. 1]

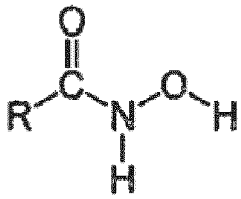

(1)

worin die Gruppe R für eine Phenylgruppe, eine Hydroxyphenylgruppe, eine Naphthylgruppe, eine Pyridylgruppe, eine aromatische Alkylgruppe mit einer Butoxygruppe, eine Alkylgruppe mit einer Benzamidstruktur oder eine Alkylgruppe mit einer Phenylcarbamoylgruppe steht,
wobei die wasserabsorbierende Harzzusammensetzung ein Gemisch aus der Hydroxamsäure-Spezies und dem wasserabsorbierenden Harz in Form von Teilchen ist und
wobei der Gehalt der Hydroxamsäure-Spezies im Bereich von 0,05 bis 1,0 Massenteilen pro 100 Massenteile des wasserabsorbierenden Harzes liegt.

**2.** Wasserabsorbierende Harzzusammensetzung nach Anspruch 1, wobei die durch die allgemeine Formel (1) dargestellte Hydroxamsäure-Spezies in Form eines Alkalimetallsalzes oder eines Ammoniumsalzes enthalten ist.

**3.** Wasserabsorbierende Harzzusammensetzung nach Anspruch 1 oder 2, wobei die Hydroxamsäure-Spezies oder das Salz davon zumindest eine/s, ausgewählt aus der aus Salicylhydroxamsäure, Benzohydroxamsäure, 2-(4-Butoxyphenyl)acetohydroxamsäure, 1-Naphthohydroxamsäure, 3-Pyridincarbohydroxamsäure, Suberoylanilidhydroxamsäure, 4-(Dimethylamino)-N-[7-(hydroxyamino)-7-oxoheptyl]benzamid und Natriumbenzohydroxamsäure bestehenden Gruppe ist.

**4.** Absorbierendes Material, das eine wasserabsorbierende Harzzusammensetzung nach einem der Ansprüche 1 bis 3 und eine hydrophile Faser umfasst.

**5.** Absorbierendes Erzeugnis, das ein zwischen einer flüssigkeitsdurchlässigen Lage und einer flüssigkeitsundurchlässigen Lage gehaltenes absorbierendes Material nach Anspruch 4 umfasst.

## Revendications

**1.** Composition de résine absorbant l'eau, comprenant :

une résine absorbant l'eau sous la forme de particules ; et
une espèce d'acide hydroxamique représentée par la formule générale (1) suivante ou un sel de métal alcalin de celui-ci ou un sel d'ammonium de celui-ci :

[Chem. 1]

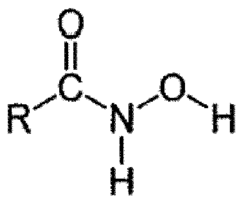

(1)

dans laquelle un groupe R représente un groupe phényle, un groupe hydroxyphényle, un groupe naphtyle, un groupe pyridyle, un groupe alkyle aromatique présentant un groupe butoxy, un groupe alkyle présentant une structure benzamide, ou un groupe alkyle présentant un groupe phénylcarbamoyle, dans laquelle la composition de résine absorbant l'eau est un mélange de l'espèce d'acide hydroxamique et de la résine absorbant l'eau sous la forme de particules, et dans laquelle la teneur de l'espèce d'acide hydroxamique est dans une plage

de 0,05 à 1,0 partie en masse pour 100 parties en masse de la résine absorbant l'eau.

**2.** Composition de résine absorbant l'eau selon la revendication 1, dans laquelle l'espèce d'acide hydroxamique représentée par la formule générale (1) est contenue sous la forme d'un sel de métal alcalin ou d'un sel d'ammonium.

**3.** Composition de résine absorbant l'eau selon la revendication 1 ou 2, dans laquelle l'espèce d'acide hydroxamique ou un sel de celui-ci est au moins un choisi dans le groupe constitué de l'acide salicylhydroxamique, de l'acide benzohydroxamique, de l'acide 2-(4-butoxyphényl)acétohydroxamique, de l'acide 1-naphthohydroxamique, de l'acide 3-pyridinecarbohydroxamique, de l'acide subéroylanilide hydroxamique, du 4-(diméthylamino)-N-[7-(hydroxyamino)-7-oxoheptyl]benzamide, et de l'acide benzohydroxamique sodique.

**4.** Matériau absorbant comprenant la composition de résine absorbant l'eau selon l'une quelconque des revendications 1 à 3 et une fibre hydrophile.

**5.** Article absorbant comprenant le matériau absorbant selon la revendication 4 maintenu entre une feuille perméable aux liquides et une feuille imperméable aux liquides.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- JP 2001258934 A
- EP 1616581 A1
- US 3920015 A
- JP S6415045 A
- WO 9826808 A2
- JP 2001037805 A
- JP 11512946 A
- JP 6051843 A
- JP 2001505237 A